# EUROPEAN PATENT APPLICATION

(11) **EP 2 335 665 A1**
(43) Date of publication of application: **22.06.2011**
(21) Application number: 09817741.3
(22) Date of filing: 28.09.2009
(51) Int. Cl.: A61F 13/15, A61F 13/49

(54) **LIQUID HANDLING ARTICLE AND WEARING ARTICLE INCLUDING THE SAME**

(30) Priority: 30.09.2008 JP 2008255493; 30.09.2008 JP 2008255494
(71) Applicant: Unicharm Corporation, Ehime 799-0111 (JP)
(72) Inventor: SAKAGUCHI, Satoru, Kanonji-shi Kagawa 769-1602 (JP)
(74) Representative: Peter, Julian
(86) International application number: PCT/JP2009/066812
(87) International publication number: WO 2010/038708

(57) **Abstract**

The present invention provides a bodily fluid containment structure that the bodily fluids once received therein should not leak out from the structure even when the wearer's weight is exerted thereon or the wearer changes his or her posture and to provide a wearing article including this containment structure.

A chassis 2 is provided on outer surface of an outer sheet 9 with a bodily fluid containment structure 15. In a crotch region 7, an inner liner 8 and an outer sheet 9 are formed with through-hole 16 extending through these sheets. Inner and outer sheets 18, 19 of the containment structure 15 are bonded to each other along outer peripheral edges thereof to form an envelope. The inner sheet 18 is formed with an opening 25 and, on both sides of this opening as viewed in a longitudinal direction Y, front and rear containment regions adapted to contain bodily fluids are formed. The opening 25 of the containment structure 15 is aligned with the through-hole 16 extending through the inner liner 8 and the outer sheet 9 and the peripheral edge of the opening 25 is bonded to the outer sheet 9 by first bonding means 49. A bonded region 28 is formed between the inner and outer sheets 18, 19 to bond these sheets to each other. The bonded region 28 comprises a plurality of bonded line segments and first channels are formed by these bonded line segments.

## Description

### TECHNICAL FIELD

The present invention relates to wearing articles including bodily fluid containment structures and more particularly to wearing articles such as disposable diapers, toilet-training pants or incontinent briefs, each including bodily fluid containment structures.

### RELATED ART

Conventionally, wearing articles incorporated with an envelope-shaped absorbent structure having an opening toward which urine is discharged are known, for example, from JP 2005-323797 A (PATENT DOCUMENT 1) . According to the disclosure, a guide sheet functioning to disperse urine is provided between the opening and a bottom of the envelope-shaped absorbent structure and side edges of the guide sheet are covered with the absorbent structure to achieve absorption of urine. A skin-contact sheet is provided between the guide sheet and the opening to protect the wearer' s skin from contact with discharged urine.

### PRIOR ART DOCUMENT

### PATENT DOCUMENT

PATENT DOCUMENT 1: JP 2005-323797A

### SUMMARY OF THE INVENTION

### PROBLEM TO BE SOLVED BY THE INVENTION

However, urine once having been absorbed in the absorbent structure may leach out therefrom, for example, the wearer's body weight is exerted thereon. In this state, if the wearer turns over on his or her side or grabbles, urine moves within the envelope-shaped absorbent structure and sometimes leaks out through the opening.

It is an object of the present invention to provide a bodily fluid containment structure that the bodily fluids once received therein should not leak out from the structure even when the wearer's weight is exerted thereon or the wearer changes his or her posture and to provide a wearing article including this bodily fluid containment structure.

### MEASURE TO SOLVE THE PROBLEM

The present invention includes first through fourth aspects.
According to the first aspect of the present invention, there is provided a bodily fluid containment structure having a longitudinal direction extending rearward from a front of the wearer's body, a transverse direction being orthogonal to the longitudinal direction, a liquid-impervious inner sheet facing the wearer's body and a liquid-impervious outer sheet facing away from the wearer's body wherein the inner and outer sheets cooperate with each other to form an envelope, the inner sheet is formed with an opening allowing bodily fluids to flow into the envelope and a bodily fluid containment region is formed between the inner and outer sheet as the inner and outer sheets are spaced from each other by inflow of bodily fluids thereinto.

The first aspect of the present invention is characterized in that a bonded region substantially bonding the inner and outer sheets to each other is formed between a peripheral edge of the opening and a peripheral edge of the envelope so that the bonded region forms first channel allowing bodily fluids to move between the opening and the containment region, and the containment region is defined between the first channel and a peripheral edge of the envelope.
As used herein, "substantially bonding the inner and outer sheets to each other" means to include not only a case in which the inner and outer sheets are directly bonded to each other but also a case in which these inner and outer sheets are bonded to each other by means of the other sheet or the like member.

Preferred embodiments according to the present invention on the first aspect thereof include an embodiment in which a peripheral edge of the envelope includes opposite side edges extending in the longitudinal direction and front and rear ends extending in the transverse direction and the bonded region includes, at least between the front and rear ends, a first non-bonded region in which the inner and outer sheets are substantially not bonded to each other and this first non-bonded region defines the containment region.

Preferred embodiments according to the present invention on the first aspect thereof include an embodiment in which a plurality of the bonded regions are formed so that the first channel is defined between at least the adjacent two bonded regions.

Preferred embodiments according to the present invention on the first aspect thereof include an embodiment in which a second channel is defined between the bonded region and at least one of the side edges and one of front and rear ends of this second channel communicates with the containment region.

Preferred embodiments according to the present invention on the first aspect thereof include an embodiment in which the envelope includes a transverse center line bisecting a dimension of the envelope in the longitudinal direction and cross-sections of the first and second channels taken along a line extending in parallel to the transverse center line respectively have circular shapes defined by at least the inner and outer sheets and the bonded region wherein a perimeter of the circular cross-section defined by at least one of the second channels is longer than a perimeter of the cross-section defined by the first channel.

Preferred embodiments according to the present invention on the first aspect thereof include an embodiment in which a plurality of the first channels are formed so as to be arranged in the transverse direction and, when the cross-sections of the first and second channels taken along a line extending in parallel to the transverse direction have perfect circular shapes, a total area of the cross-section defined by the second channel is larger than a total area of the cross-sections defined by the first channels.

Preferred embodiments according to the present invention on the first aspect thereof include an embodiment in which the bonded region is formed by at least linear bonded line segments.

Preferred embodiments according to the present invention on the first aspect thereof include an embodiment in which the first channels are defined by the bonded line segments spaced one from another by the narrowest distances.

Preferred embodiments according to the present invention on the first aspect thereof include an embodiment in which the liquid-absorbent structure adapted to absorb bodily fluids and to contain this is formed between the inner and outer sheets.

Preferred embodiments according to the present invention on the first aspect thereof include an embodiment the liquid-absorbent structure is formed in the containment region.

Preferred embodiments according to the present invention on the first aspect thereof include an embodiment in which a second non-bonded region in which the inner and outer sheets are substantially not bonded to each other is formed between the opening and the bonded region.

According to the present invention on the second aspect thereof, there is provided a wearing article comprising a chassis having a longitudinal direction, a transverse direction, a side facing the wearer' s body and a side facing away from the wearer' s body, a front waist region, a rear waist region and a crotch region extending between the front and rear waist regions wherein these regions are contiguous one to another in the longitudinal direction.
The second aspect of the present invention is characterized in that the wearing article further comprises a bodily fluid containment structure described in the first aspect of the present invention at least in said crotch region and the opening of the bodily fluid containment structure lies in said crotch region.

Preferred embodiments according to the present invention on the second aspect thereof include an embodiment in which the bodily fluid containment structure is formed on the side of the chassis facing away from the wearer's body and the chassis is formed at least in the crotch region so as to allow bodily fluids to flow into the opening of the bodily fluid containment structure.

Preferred embodiments according to the present invention on the second aspect thereof include an embodiment in which the chassis is formed in the crotch region with a through-hole through which bodily fluid can flow into the opening of the bodily fluid containment structure via the through-hole.

According to the present invention on the third aspect thereof, there is provided a bodily fluid containment structure having a longitudinal direction extending rearward from a front of the wearer's body, a transverse direction being orthogonal to the longitudinal direction, comprising a liquid-impervious inner sheet facing the wearer's body and a liquid-impervious outer sheet facing away from the wearer's body wherein the inner and outer sheets cooperate with each other to form an envelope, the inner sheet is formed with an opening allowing bodily fluids to flow into the envelope and a bodily fluid containment region is formed between the inner and outer sheet as the inner and outer sheets are spaced from each other by inflow of bodily fluids thereinto.

The third aspect of the present invention is characterized in that a backflow barrier sheet covering the opening is provided between the inner and outer sheet and the backflow barrier sheet is intermittently bonded to the inner sheet by means of a bonded region so that the containment region may be formed between the backflow barrier sheet and the outer sheet also.

Preferred embodiments according to the present invention on the third aspect thereof include an embodiment in which a plurality of the bonded regions and the rear channel making the opening fluid-communicate with the containment region is formed at least a pair of the bonded regions being adjacent in the transverse direction.

Preferred embodiments according to the present invention on the third aspect thereof include an embodiment in which the backflow barrier sheet side edges extending in the longitudinal direction and front and rear ends extending in the transverse direction wherein an outer side non-bonded region in which the backflow barrier sheet and the inner sheet are substantially not bonded to each other is formed between the bonded region and at least one of the side edges or the front and rear ends of the backflow barrier sheet.

Preferred embodiments according to the present invention on the third aspect thereof include an embodiment in which the bonded region is formed at least by bonded line segments.

Preferred embodiments according to the present invention on the third aspect thereof include an embodiment in which a distance between each pair of the adjacent bonded line segments is gradually reduced as these line segments get closer to the containment region.

Preferred embodiments according to the present invention on the third aspect thereof include an embodiment in which the containment region defined at least between the inner and outer sheets is formed with the liquid-absorbent structure adapted to absorb and to contain bodily fluids.

Preferred embodiments according to the present invention on the third aspect thereof include an embodiment in which the liquid-absorbent structure is formed in the containment region defined between the inner and outer sheets and in the containment region defined between the backflow barrier sheet and the outer sheet.

Preferred embodiments according to the present invention on the third aspect thereof include an embodiment in which the inner side non-bonded region in which the inner sheet and the backflow barrier sheet are substantially not bonded to each other is formed between the opening and the bonded region.

Preferred embodiments according to the present invention on the third aspect thereof include an embodiment in which the inner sheet comprises a first inner sheet lying on the side facing the wearer's body and a second inner sheet lying on the side facing away from the wearer's body wherein the backflow barrier sheet is bonded to the second inner sheet by means of the bonded region.

According to the present invention on the fourth aspect thereof, there is provided a wearing article comprising a chassis having a longitudinal direction and a transverse direction, a side facing the wearer's body and a side facing away from the wearer's body, a front waist region, a rear waist region and a crotch region extending between the front and rear waist regions wherein these regions are contiguous one to another in the longitudinal direction.

The fourth aspect of the present invention is characterized in that the wearing article comprises the bodily fluid containment structure defined in the description of the third aspect of the present invention at least in said crotch region and the opening of said bodily fluid containment structure lies in the crotch region.

Preferred embodiments according to the present invention on the fourth aspect thereof include an embodiment in that the bodily fluid containment structure is formed on the side facing away from the wearer's body and the chassis is adapted, at least in the crotch region thereof, to allow inflow of bodily fluids into the opening of the bodily fluid containment structure.

Preferred embodiments according to the present invention on the fourth aspect thereof include an embodiment in which said chassis is formed in the crotch region thereof with a through-hole overlapping the opening of the bodily fluid containment structure so that bodily fluids may flow into the opening.

Preferred embodiments according to the present invention on the fourth aspect thereof include an embodiment in which the through-hole has an opening area dimensioned to be smaller than an opening area of the opening of the bodily fluid containment structure.

### EFFECT OF THE INVENTION

According to the first aspect of the present invention, the bodily fluid containment structure includes the bonded region in which the inner and outer sheets are bonded to each other. Each pair of the adjacent sub-regions of the bonded region cooperates with each other to form the first channel. The first channel serves to force bodily fluids coming from the opening and intending to pass through the first channel to be dispersed toward the containment region. In consequence, the backflow of bodily fluids toward the opening can be restricted and thereby leak of bodily fluids such as urine through the opening can be restricted.

In the wearing article according to the second aspect of the present invention, the bodily fluid containment structure is located at least in the crotch region so that the opening of the envelope-shaped containment region. With this arrangement, for example, when the diaper is used in combination with the containment structure, discharged urine can be contained in the envelope-shaped containment structure. In this way, urine leakage out of the wearing article can be restricted.

According to the third aspect of the present invention, the bodily fluid containment structure includes the inner and outer sheets between which the backflow barrier sheet is sandwiched so as to cover the opening. With this arrangement, it is possible to restrict urine leakage out of the containment region through the opening. The backflow barrier sheet is intermittently bonded to the inner sheet by means of the bonded region so that bodily fluids can flow into the containment region through the opening.

In the wearing article according to the fourth aspect of the present invention, the bodily fluid containment region is located at least in the crotch region so that the opening of the bodily fluid containment region lies in the crotch region. With such arrangement, discharged urine can be contained in the envelope of the bodily fluid containment structure, for example, during use of the wearing article such as diaper. In consequence, the urine leakage out of the wearing article can be restricted.

### BRIEF DESCRIPTION OF THE DRAWINGS

[FIG. 1]

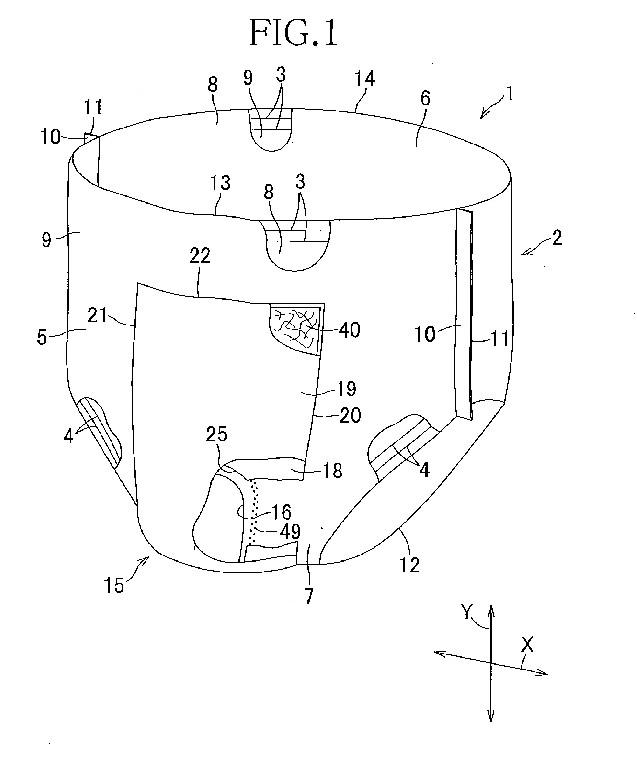

   Fig. 1 is a perspective view of a diaper as one example of wearing articles.
[FIG. 2]

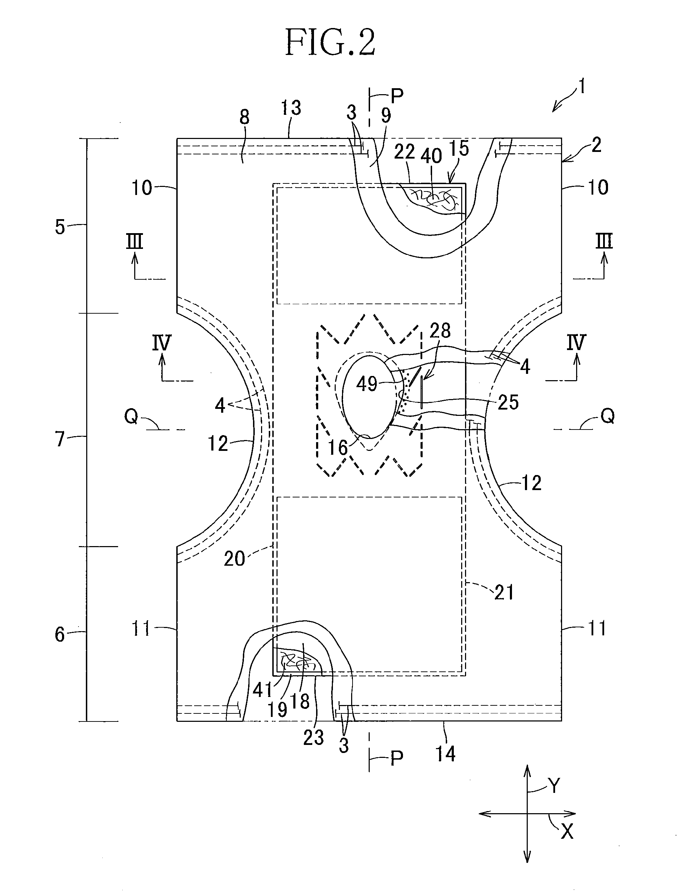

   Fig. 2 is a plan view of the diaper as has been flatly developed.
[FIG. 3]

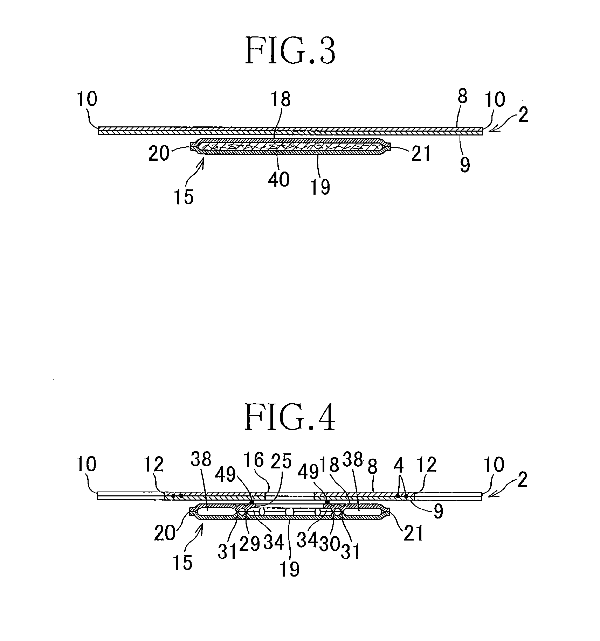

   Fig. 3 is a sectional view taken along line III-III in Fig. 2.
[FIG. 4]
   Fig. 4 is a sectional view taken along line IV-IV in Fig 2.
[FIG. 5]

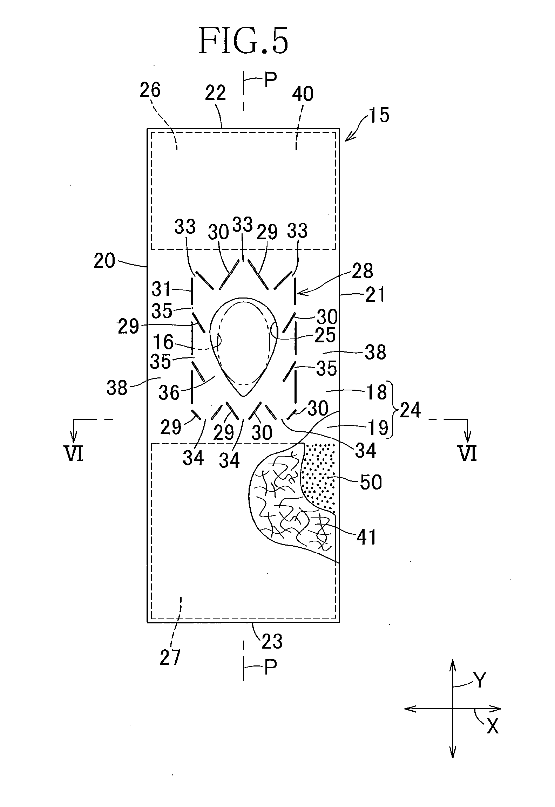

   Fig. 5 is a plan view of a bodily fluid containment structure.
[FIG. 6]

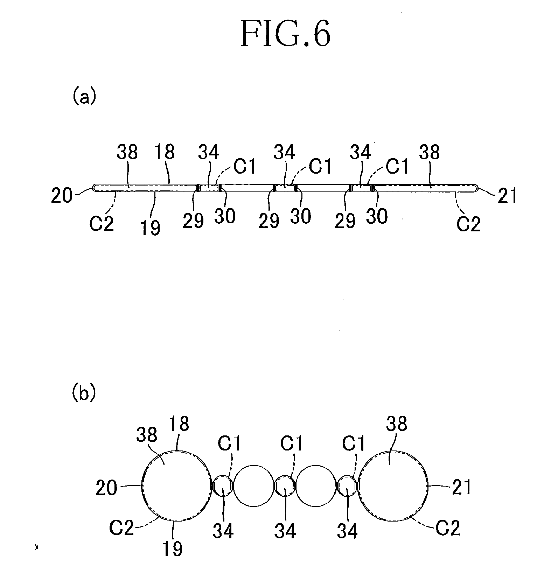

   Fig. 6 (a) is a sectional view taken along line VI-VI in Fig. 5 and Fig. 6 (b) is a schematic view similar to Fig. 6 (a).
[FIG. 7]

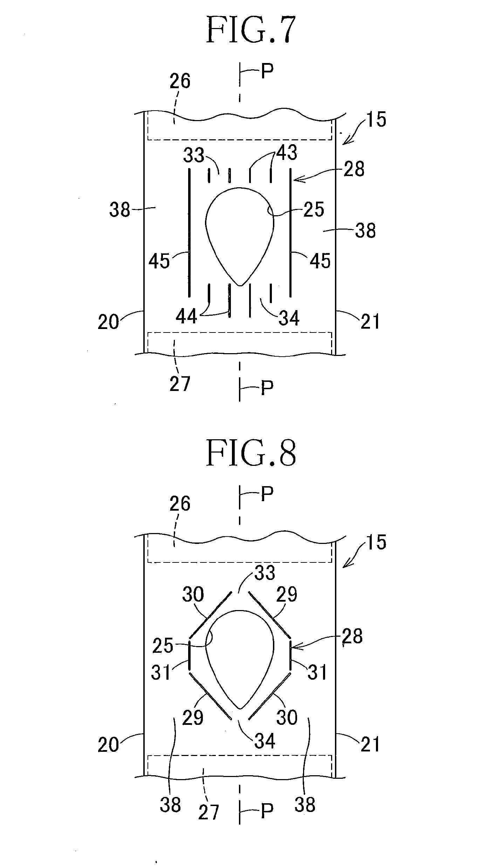

   Fig. 7 is a plan view of another embodiment of the containment structure.
[FIG. 8]
   Fig. 8 is a plan view of still another embodiment of the containment structure.
[FIG. 9]

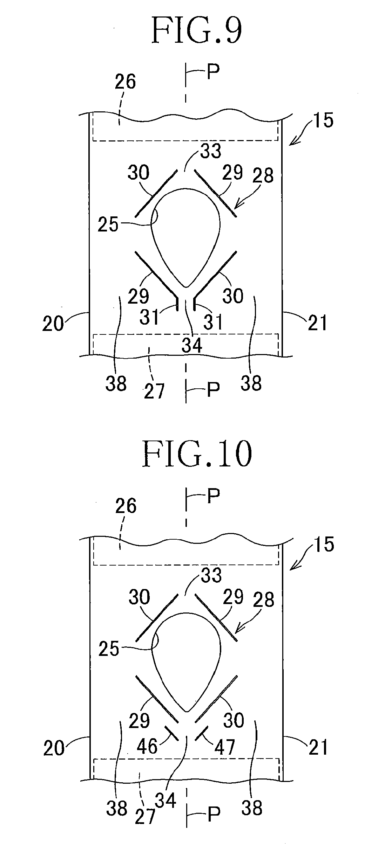

   Fig. 9 is a plan view of yet another embodiment of the containment structure.
[FIG. 10]
   Fig. 10 is a plan view of further another embodiment of the containment structure.
[FIG. 11]

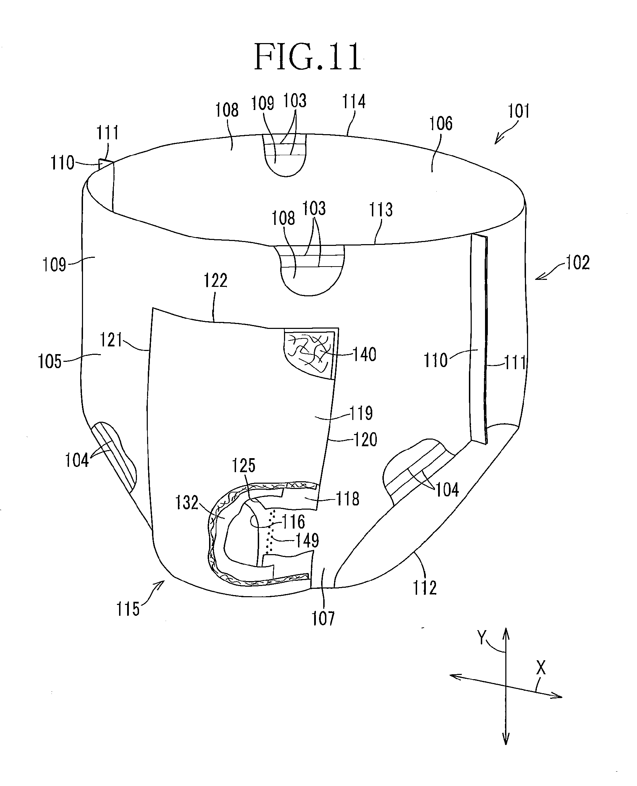

   Fig. 11 is a perspective view of the diaper.
[FIG. 12]

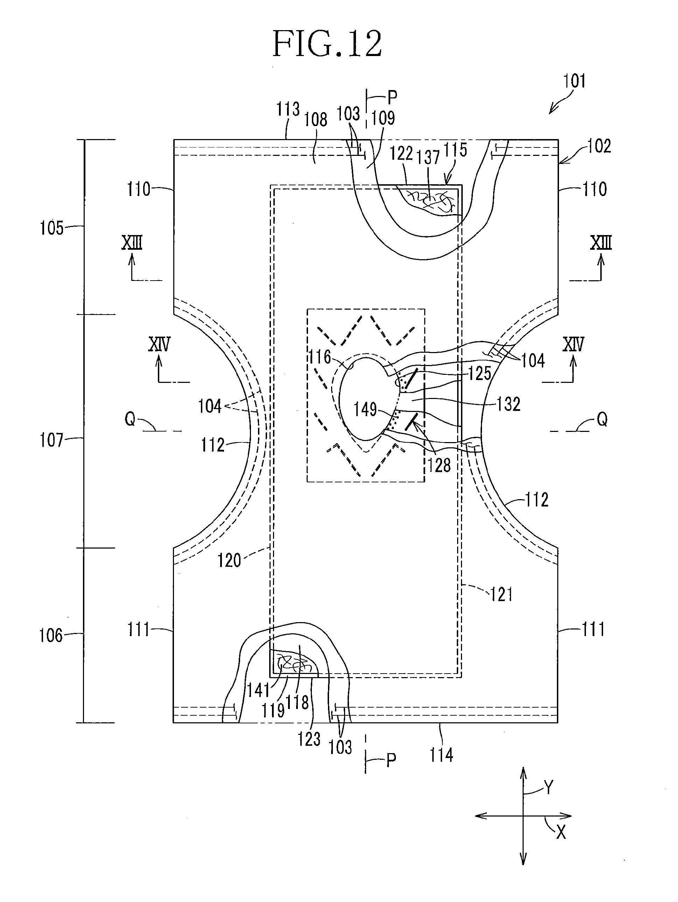

   Fig. 12 is a plan view of the diaper as has been flatly developed.
[FIG. 13]

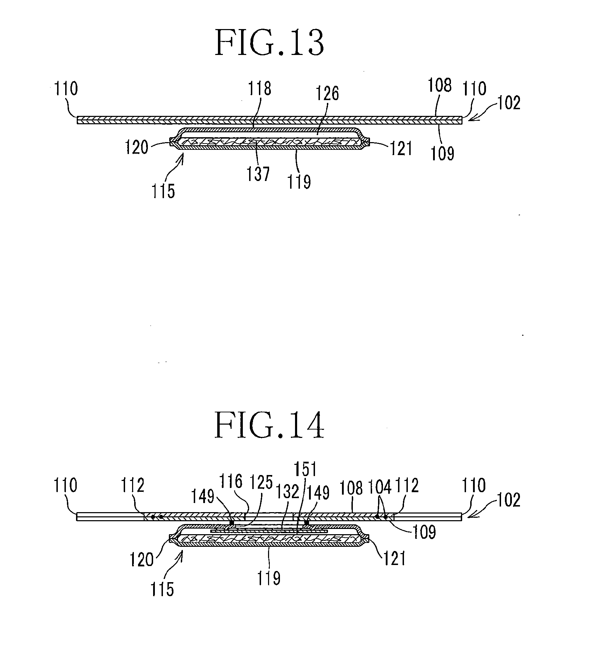

   Fig. 13 is a sectional view taken along line XIII-XIII in Fig. 12.
[FIG. 14]
   Fig. 14 is a sectional view taken along line XIV-XIV in Fig. 12.
[FIG. 15]

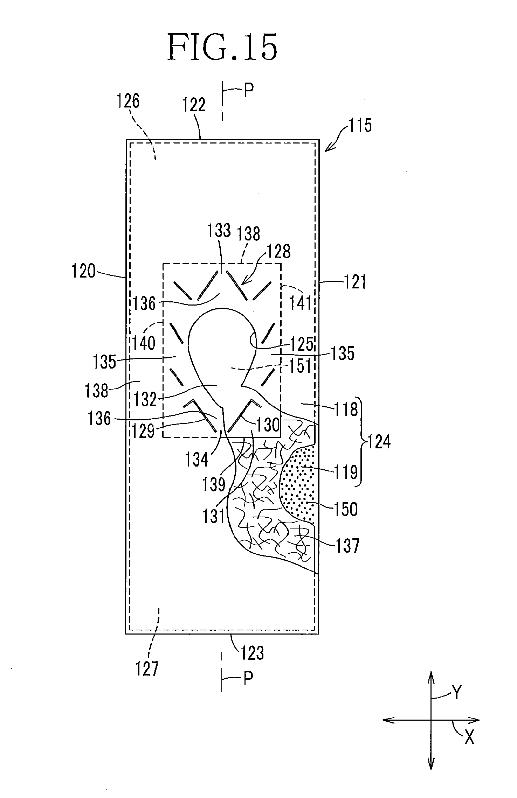

   Fig. 15 is a plan view of the containment structure.
[FIG. 16]

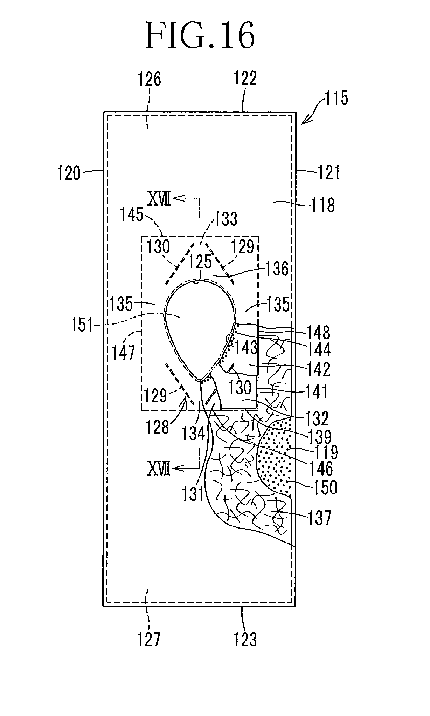

   Fig. 16 is a plan view of the containment structure according to the other embodiment.
[FIG. 17]

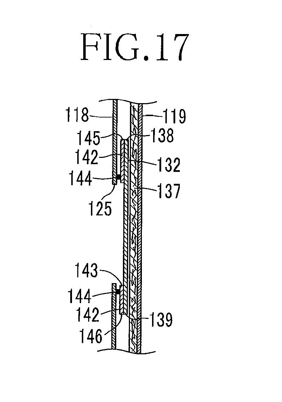

   Fig. 17 is a sectional view taken along line XVII - XVII in Fig. 16.

### DETAILED DESCRIPTION OF THE PREFERRED EMBODIMENTS

Details of the present invention on first and second aspects thereof will be more fully understood from the description made hereunder based on embodiments exemplarily described wherein a disposable diaper is taken as one example of wearing articles.

Fig. 1 shows the diaper 1 formed in a pants-type and Fig. 2 is a plan view of the diaper 1 in a flatly developed state after front and rear waist regions having been peeled off each other along both side edges of the diaper 1. In Figs. 1 and 2, the diaper 1 is shown in partially cutaway views for convenience of explanation. In Fig. 2, contraction of respective elastic members is restricted so that the diaper 1 may be kept in its developed state. Fig. 3 is a sectional view taken along line III-III in Fig. 2 and Fig. 4 is a sectional view taken along line IV-IV in Fig. 2. The diaper 1 includes a chassis 2, waist elasticmembers 3 and leg elasticmembers 4. The chassis 2 comprises a front waist region 5, a rear waist region 6 and a crotch region 7 extending between these front and rear waist regions 5, 6. These regions 5, 6, 7 are contiguous one to another in a longitudinal direction Y. In addition, the chassis 2 has a longitudinal center line P-P bisecting a dimension of the diaper 1 in a transverse direction X and a transverse center line Q-Q bisecting a dimension of the diaper 1 in the longitudinal direction Y.

The chassis 2 includes an inner liner 8 defining a side of the chassis 2 facing the wearer's body and an outer sheet 9 defining an opposite side of the chassis 2 facing away from the wearer's body. The waist elastic members 3 and the leg elastic members 4 are sandwiched between these inner liner 8 and outer sheet 9. Both the waist elastic members 3 and the leg elastic members 4 are secured to at least one of the inner liner 8 and the outer sheet 9. The chassis 2 includes front waist side edges 10 extending in the longitudinal direction Y, rear waist side edges 11 extending in the longitudinal direction and crotch side edges 12.

The chassis 2 includes front and rear ends 13, 14 extending in the transverse direction X, i.e., extending orthogonally to the longitudinal direction Y. The crotch side edges 12 are curved in the crotch region 7 toward the longitudinal center line P-P and the leg elastic members 4 extend along the respective crotch side edges 12 and are attached under tension to the respective crotch side edges 12. The waist elastic members 3 extend along the front and rear ends 13, 14 of the chassis 2 and are contractibly attached under tension to the respective crotch side edges 12. The font waist side edges 10 and the rear waist side edges 11 are joined together to connect these front and rear waist regions 5, 6 with each other and to form the diaper 1 of pants-type.

A bodily fluid containment structure 15 is attached to the outer side of the outer sheet 9. The bodily fluid containment structure 15 is placed at least in the crotch region 7 and extends into the front and rear waist regions 5, 6 in the longitudinal direction Y. In the crotch region 7, these inner liner 8 and outer sheet 9 are formed with a through-hole 16 extending through them. Through this through-hole 16, the bodily fluid containment structure 15 is exposed. The through-hole 16 is formed on the side of the front waist region 5 with respect to the transverse center line Q-Q and in alignment with the wearer's external genital. In other words, the wearer's external genital may be aligned with the bodily fluid containment structure 15 via the through-hole 16.

Fig. 5 is a partially cutaway plan view of the containment structure 15 partially cutaway for convenience of explanation. As shown, the containment structure 15 includes rectangular inner and outer sheets 18, 19 both of which are substantially the same in size and shape. These inner and outer sheets 18, 19 are bonded to each other along opposite side edges 20, 21 extending in the longitudinal direction Y and front and rear ends 22, 23 extending in the transverse direction X, respectively. In other words, the inner and outer sheets 18, 19 are bonded to each other along outer peripheries thereof to form an envelope 24. The inner and outer sheets 18, 19 may be formed of liquid-impervious film to make the envelope 24 as a whole liquid-impervious. The inner sheet 18 is formed with an opening 25. Within the envelope 24, front and rear containment regions 26, 27 adapted to contain bodily fluids are defined in the front and in the rear of the opening 25 in the longitudinal direction Y and the aforementioned opposite side edges 20, 21 cooperate with the aforementioned front and rear ends 22, 23 to define the peripheral edge of the envelope 24.

The inner sheet 18 of the containment structure 15 lies on the side facing the wearer's body and is bonded to the outer sheet 9 of the chassis 2. The opening 25 of the containment structure 15 is formed so as to be aligned with the through-hole 16 formed to as to extend through the inner liner 8 and the outer sheet 9. A peripheral edge of the opening 25 is bonded to the outer sheet 9 by first bonding means 49 (See Figs. 1 and 2). The first bonding means 49 is formed along the peripheral edge of the opening 25 to prevent the opening 25 and the through-hole 16 from being separated from each other. In consequence, the wearer's external genital aligned with the through-hole 16 is surely aligned with the opening 25 of the bodily fluid containment structure 15 so that discharged urine is reliably guided through the opening 25 into the envelope 24. The through-hole 16 and the opening 25 may be appropriately dimensioned to receive the wearer's external genital.

Between the inner and outer sheets 18, 19, bonded regions 28 in which these two sheets 18, 19 are bonded, are formed outside the first bonding means 49 so that the bonded regions 28 do not overlap the first bonding means 49 and is defined by a plurality of bonded line segments formed between the peripheral edge of the opening 25 and the peripheral edge of the envelope 24. More specifically, the bonded regions 28 are defined by first bonded line segments 29 and secondbonded line segments 30 both diagonally extending with respect to the longitudinal center line P-P and third bonded line segments 31 extending generally in parallel to the longitudinal center line P-P. The first bonded line segment 29 extend diagonally right down and the secondbonded line segments 30 extend diagonally right up. These first bonded line segments 29 and the second bonded line segments 30 are arranged alternately in the transverse direction X.

The first bonded line segments 29, the second bonded line segments 30 and the third bonded line segments 31 are respectively spaced from the side edges 20, 21 as well as from the front and rearends22, 23 of the envelope 24. Specifically, first non-bonded regions are defined between the first to thirdbonded line segments 29 to 31 and the front and rear ends 22, 23 and these first non-bonded regions respectively define the front and rear containment regions 26, 27. On the side of the front containment region 26, the first bonded line segment 29 cooperates with the second bonded line segment 30, the first bonded line segment 29 cooperates with the third bonded line segment 31 and the second bonded line segment 30 cooperates with the third bonded line segment 31, respectively, to define three funnel-shaped front side first channels 33 tapered from the opening 25 toward the front containment region 26. On the side of the rear containment region 27, the first bonded line segments 29 cooperate with the second bonded line segments 30 to define three funnel-shaped rear side first channels 34 tapered from the opening 25 toward the rear containment region 27.

Between the opening 25 and the side edges 20, 21, the third bonded line segments 31 are intermittently arranged in the longitudinal direction Y. On the side of the side edge 20, the first bonded line segment 29 is formed between each pair of the adjacent third bonded line segments 31 and, on the side of the side edge 21, the second bonded line segment 30 is formed between each pair of the adjacent third boned line segments 31, the second bonded line segment 30 is formed. The first bonded line segments 29 cooperate with the third bonded line segments 31 and the second bonded line segments 30 cooperate with the third bonded line segments 31, respectively, to define lateral first channels 35.

Between the opening 25 and the first to third bonded line segments 29 to 31, a second non-bonded region 36 is defined. In this second non-bonded region 36, the inner and outer sheets 18, 19 merely overlap each other but not bonded to each other. This second non-bonded region 36 is formed so as to overlap the first bonding means 49. Outside the front and rear side first channels 33, 34 as viewed in the transverse direction X, second channels 38 are defined and via these second channels 38, the front and rear containment regions 26, 27 communicate with each other. A second dimension of the respective second channels in the transverse direction X is larger than a first dimension of the respective front and rear side first channels 33, 34 in the transverse direction X. The second channels 38 are formed by the outermost first channels 33, 34 as viewed in the transverse direction X and the both side edges 20, 21. While a dimension from the respective side edges 20, 21 to the front side first channel 33 is not equal to a dimension from the respective side edges 20, 21 to the rear side first channel 34 and, in consequence, the second dimension of the respective second channels 38 is not the same on the front side and on the rear side according to the present embodiment, it is preferable in any case to assure that the second dimension of the respective second channels 38 is larger than the first dimension of the front and rear side first channels 38.

The front and rear containment regions 26, 27 are formed with front and rear liquid-absorbent structures 40, 41, respectively. Each of the front and rear liquid-absorbent structures 40, 41 comprises a liquid-absorbent core formed of superabsorbent resin particles and/or fluff pulp fibers and a liquid-dispersant sheet such as tissue paper with which the liquid-absorbent core is wrapped. These front and rear liquid-absorbent structures 40, 41 are bonded to the outer sheet 19 by second bonding means 50, respectively. The rear liquid-absorbent structure 41 has an area larger than an area of the front liquid-absorbent structure 40. With the diaper 1 put on the wearer's body, the front containment region 26 lies on the wearer's ventral side and the rear containment region 27 lies on the wearer's crotch and dorsal side. The discharged urine necessarily flows toward the rear containment region 27 when the wearer is in upright posture. To deal with this, the rear liquid-absorbent structure 41 is dimensioned to be relatively large and thereby the absorption capacity is increased.

In the aforementioned arrangement, upon urination occurring toward the opening 25, the inner and outer sheets 18, 19 are spaced from each other under urine pressure and the discharged urine is temporarily contained in the second non-bonded region 36. If the wearer is in upright posture, the urine is forced to flow from the second non-bonded region 36 downward to the crotch region and the quantity having been temporarily contained flows principally toward the rear side first channels 34. The rear side first channels 34 are defined by the first and second bonded line segments 29, 30 in funnel-shapes, respectively, the urine having been temporarily contained in the second non-bonded region 36 is collected by and passing through the rear side first channels 34. In the course of passage of urine through the rear side first channels 34, the inner and outer sheets 18, 19 are spaced from each other to form tubular channels. The urine having passed through the rear side first channels 34 is then guided to the rear containment region 27 and is absorbed and contained by the rear liquid-absorbent structure 41 in the rear containment region 27.

Fig. 6 (a) is a partially cutaway sectional view taken along line VI-VI in Fig. 5 and Fig. 6 (b) is a diagram schematically illustrating how this sectional view is changed after the inner and outer sheets 18, 19 have been spaced from each other. As illustrated, on both sides of the rear side first channels 34 in the transverse direction X, the second channels 38 respectively having the second dimension larger than the first dimension of the respective rear side first channels 34 are formed.
The rear side first channel 34 defines a circular cross-section C1 and the second channel 38 defines a circular cross-section C2.

A perimeter of the circular cross-section C1 formed by the rear side first channel 34 is defined by a sum of respective thickness dimensions of the first and second bonded line segments 29, 30 and respective length dimensions of the inner and outer sheets 18, 19 extending between these first and second bonded line segments 29, 30 in the transverse direction X. A perimeter of the circular cross-section C2 formed by the second channel 38 is defined by a sum of a thickness dimension of the first or second bonded line segment 29 or 30 and respective length dimensions of the inner and outer sheets 18, 19 extending from the first or second bonded line segment 29 of 30 to the side edge 20 or 21 in the transverse direction X. The second dimension of the second channel 38 defined by the length dimensions of the inner and outer sheets 18, 19 in the transverse direction X may be set to be larger than the first dimension of the rear side first channel 34 defined by the length dimensions the inner and outer sheets 18, 19 in the transverse direction X to assure that the perimeter of the circular cross-section C2 formed by the second channel 38 is larger than the perimeter of the cross-section C1 formed by the rear side first channel 34.

Fig. 6 (b) illustrates a case in which the inner and outer sheets 18, 19 are spaced from each other and the rear side first channels 34 as well as the second channels 38 form perfect circular cross-sections. Assumed that the rear side first channels 34 and the second channels 38 are deformed to have perfect circular cross-sections, the dimensional relation between the first channels 34 and the second channels 38 are previously regulated so that a total area of the circular cross-sections C2 formed by the two second channels 38 is larger than a total area of the circular cross-sections C1 formed by the three rear side first channels 34.

In the aforementioned diaper 1, the front and rear first channels 33, 34 are adapted to guide urine into the front and rear containment regions 26, 27. The flow of urine moves into the containment structure and then passes through the front and rear first channels 33, 34 into the front and rear containment regions 26, 27. Immediately after having passed through the front and rear first channels 33, 34, urine is dispersed over the front and rear containment regions 26, 27 both of which have relatively large areas. Such dispersion serves to restrict the backflow of urine through the front and rear side first channels 33, 34 toward the opening 25 and thereby to restrict urine leakage out of the diaper 1.

Front and rear ends of the second channels 38 may be put in fluid-communication with the front and rear containment regions 26, 27, respectively, and the aforementioned dimensional relationship between the rear side first channels and the second channels 38 may be satisfied to assure that the flow of urine is preferentially guided through the second channels 38 even when urine having been not absorbed by the liquid-absorbent structures moves from the rear containment region 27 toward the front containment region 26. Change of the wearer's posture, for example, from an upright posture to a side lying posture or to a groveling posture may cause the flow of urine to move from the rear containment region 27 to the front containment region 26. In this case, however, the movement of urine between the front and rear containment regions 26, 27 occurs primarily via the second channels 38 and not via the first channels. Consequentially, urine leakage out of the opening via the first channels can be restricted.

This is true for the movement of urine from the front containment region 26 to the rear containment region 27 and urine leakage out of the opening 25 via the front side first channels 33 can be reliably restricted.
The front containment region 26 is formed with the front side liquid-absorbent structure 36 and, when the urine movement from the rear containment region 27 to the front containment region 26, urine having been not absorbed by the rear side liquid-absorbent structure 37 is absorbed and contained by the front side liquid-absorbent structure 36. In this way, urine leakage out of the opening 25 can be further reliably restricted.

After the flow of urine has passed through the opening 25 and the rear side first channels 34 into the rear containment region 27, the inner and outer sheets 18, 19 are put in contact again with each other. Specifically, the tubular channels collapse and lose the function thereof as the channels. As a result, it is difficult for the flow of urine moving from the rear containment region 27 to the front containment region 26 to pass through the rear side first channels 34. In this way, the backflow of urine via the rear side first channels 34 toward the opening can be restricted.

The funnel-shaped front and rear side first channels 33, 34 are formed by the first and second bonded line segments 29, 30 defining the bonded region 28 extending diagonally right up and right down, respectively, and these funnel-shaped channels 33, 34 can efficiently collect and guide the flow of urine discharged into the opening 25 toward the front and rear containment regions 26, 27. Even if the front and rear side first channels 33, 34 are relatively narrow, a large quantity of urine can be guided by these channels 33, 34 into the front and rear containment regions 26, 27. By dimensioning the width of the front and rear side first channels 33, 34 to be relatively narrow, a difference between a total area of the first channels 33, 34 and a total area of the second channels 35 can be increased and the flow of urine can be preferentially guided through the second channels 38. In this way, the passage of urine through the front and rear side first channels 33, 34 can be restricted. In consequence, urine leakage out of the opening 25 via the front and rear side first channels 33, 34 can be further reliably restricted.

The funnel-shaped front and rear side first channels 33, 34 facilitate the flow of urine moving from the opening 25 toward the front and rear containment regions 26, 27 to be collected and forces the flow of urine moving from the front and rear containment regions 26, 27 toward the opening 25 to be dispersed back over the front and rear containment regions 26, 27. In consequence, urine passing through the front and rear side first channels 33, 34 can be minimized and urine leakage out of the opening 25 can be more reliably restricted.

According to the present embodiment, discharged urine can be contained in the bodily fluid containment structure 15 combined with the diaper 1 as the wearing article and there is no possibility that the urine once having been contained in the containment structure 15 might leak out of the envelope 24 unless urine leaks out through the opening. Specifically, the discharged urine is absorbed and contained by the containment structure 15 provided separately of the wearing article and therefore urine leakage out of the diaper 1 as the wearing article can be restricted at the highest reliability. In addition, the separately prepared envelope adapted to receive the discharged urine allows a relatively large quantity of urine to be contained within the envelope and thereby makes it unnecessary to exchange the diaper 1 at short intervals. In other words, the diaper 1 can be maintained put on the wearer's body for a long period.

The inner and outer sheets 18, 19 of the containment structure 15 may be formed, for example, by a liquid-impervious film and the inner liner 8 as well as the outer sheet 9 may be formed, for example, of a liquid-pervious fibrous nonwoven fabric. Each of these sheets may be formed of a single sheet or two or more sheets laminated together.
While the containment structure 15 is formed with only one opening 25 according to the present embodiment, for example, the rear containment region 27 may be formed with opening independently of the opening 25. In this case, this additional opening may be used to receive feces. While the bonded region is defined by a plurality of rectilinear line segments according to the present embodiment, the bonded region may comprise a plurality of curved line segments. It is not essential to provide the bonded region in the form of the line segments and dots or a combination of dots and line segments may be adopted to define the bonded region. In any case, it is preferable to dimension the first channels to be narrower than the second channels. The number of segments defining the bonded region 28 may be increased to reduce a width of the respective first channels and thereby passage of bodily fluids through the first channels can be further reliably restricted. The bonded region 28 may be formed by conditionally used well known means such as adhesives, thermal or ultrasonic bonding techniques. While the pullon pants-type diaper is exemplarily illustrated as the wearing article, the present invention is applicable also to a so-called open-type diaper having front and rear waist regions adapted to be joined together along opposite side edges just before the diaper is put on the wearer's body.

While the bonded region 28 is formed by directly bonding the inner and outer sheets 18, 19 to each other according to the present embodiment, it is possible to bond the inner and outer sheets 18, 19 with the other member such as a sheet. When the inner and outer sheets 18, 19 are bonded with the other means, a distance between the inner and outer sheets 18, 19 can be increased and the perimeters of the circular cross-sections formed by the first and second channels may be correspondingly increased.
While the peripheral edge of the envelope 24 is formed by bonding the side edges 20 and the front and rear ends 22, 23 of the inner sheet 18 to those of the outer sheet 19 according to the present embodiment, the position along which the inner and outer sheets 18, 19 are bonded together is not limited to the side edges 20, 21 and the front and rear ends 22, 23 so far as the position of bonding is outside the bonded region 28. Specifically, the inner and outer sheets 18, 19 may be bonded to each other along a region defined between the bonded region 28,, and the side edges 20, 21 and the front and rear ends 22, 23 to form the peripheral edge.

Figs. 7 through 10 show the containment structures 15 according to the other embodiments of the present invention. The embodiments are similar to the previous embodiment except the bonded region 28 and detailed description of the similar component elements will be hereunder eliminated.
In the embodiment shown in Fig. 7, a plurality of bonded line segments 43 forming the front side first channels 33, a plurality of bonded line segments 44 forming the rear side first channels 34 and a plurality of bonded line segments 45 forming the second channels 38 wherein all of these bonded line segments extend generally in parallel to the longitudinal center line P-P. The bonded line segments 43 are arranged in the transverse direction X generally at regular intervals and each of these bonded line segments 43 has a dimension in the longitudinal direction Y longer than the bonded line segments 43. The length dimension of bonded line segments 44 gradually becomes longer as these line segments 44 get closer to the longitudinal center line P-P. Each of the boned line segments 45 has a length dimension larger than a dimension of the opening 25 in the longitudinal direction Y. These bonded line segments 45 cooperate with the side edges 20, 21 to define the second channels 38. The second dimension of each of the second channels 38 in the transverse direction X larger than the first dimension of each of the front and rear side first channels 33, 34.

Each of the bonded line segments 44 defining the rear side first channels 34 has its dimension in the longitudinal direction Y gradually becomes longer as it gets closer to the longitudinal center line P-P according to the present embodiment and the flow resistance of the channels formed by these bonded line segments correspondingly increases. The flow of urine preferentially moves through the channels having a low resistance, i.e., through the second channels 38 each having larger dimension in the transverse direction X and substantially not through the rear side first channels 34 having a high flow resistance. According to the present embodiment, the second channels 38 have the lowest flow resistance and the flow resistance gradually increases in the front side first channels 33 and the rear side first channels 34 in this order. With respect to the rear first channels 34, the flow resistance gradually increases from the outermost sides as viewed in the transverse direction X toward the longitudinal center line P-P. Consequently, when a flow of urine moves between the front and rear containment regions 26, 27, the flow of urine preferentially moves through the second channels 38 and it is difficult for the flow of urine to move through the front and rear side first channels 33, 34.

According to the present embodiment, none of the lateral first channels is formed and the bonded line segments 45 extend in parallel to each other in the longitudinal direction Y on both sides of the opening 25. With this arrangement, even when the flow of urine moves in the transverse direction X, the bonded line segments 45 effectively serve to restrict urine leakage out of the opening 25. While each of the bonded line segments 44 is dimensioned to become longer as it gets close to the longitudinal center line P-P according to the present embodiment, such gradual variation of the length is not essential and it is possible to dimension these line segments 44 to be generally uniform. It is also possible to dimension the bonded line segments 43 and the bonded line segments 44 to have the same length. It should be noted, however, that each of the bonded line segments 44 is preferably dimensioned to become gradually longer as it gets close to the longitudinal center line P-P. This is because the flow resistance correspondingly increases as the channels get close to the longitudinal center line P-P and it becomes more difficult for the flow of urine to backflow toward the opening 25 formed on the longitudinal center line P-P.

In an embodiment shown in Fig. 8, the front side first channel 33 is formed by the first bonded line segment 29 and the second bonded line segment 30 lying on the side of the front containment region 26 and the rear side first channel 34 is formed by the first bonded line segment 29 and the second bonded line segment 30 lying on the side of the rear containment region 27. Between the front side first and second bonded line segments 29, 30 and the rear side first and second bonded line segments 29, 30, the third bonded line segments 31 extend in the longitudinal direction Y to connect the front and rear side first and second bonded line segments 29, 30, respectively. According to the present embodiment, the second channels 38 are defined outside the front and rear first channels 33 in the transverse direction X, more specifically, between the front side first channel 33 and the both side edges 20, 21 and between the rear side first channel 34 and the both side edges 20, 21, respectively.

Provision of the third bonded line segments 31 means that none of the lateral side first channels is not formed on both sides of the opening 25 as viewed in the transverse direction X. In consequence, urine leakage out of the opening 25 can be prevented even when the flow of urine moves in the transverse direction X. It should be appreciated, however, that it is not essential to provide these third bonded line segments 31 and it may be appropriately selected whether these third bonded line segments 31 are provided or not.

In an embodiment shown in Fig. 9, the front side first channel 33 is defined by the first and second bonded line segments 29, 30 lying on the side of the front containment region 26 of the containment structure 15 and the rear side first channel 34 is defined by the first and second bonded line segments 29, 30 lying on the side of the rear containment region 27 and the third bonded line segments 31 extending in the longitudinal direction Y from the associated first and second bonded line segments 29, 30 toward the rear containment region 27.

The second channels 38 are defined between the third bonded line segments 31 defining the rear side first channel 34 and the both side edges 20, 21.
The third bonded line segments 31 longitudinal direction Y and cooperating with the first and second bonded line segments 29, 30 to define the rear side first channel 34 contribute to increase a length of the channel and thereby to increase the flow resistance of the rear side first channel 34. As a result, the movement of urine flow through the rear side first channel 34 can be restricted.

In an embodiment shown in Fig. 10, the front side first channel 33 is defined by the first and second bonded line segments 29, 30 on the side of the front containment region 26 of the containment structure 15 and the rear side first channel 34 is defined by the first and second bonded line segments 29, 30 lying on the side of the rear containment region 27 and first and second bonded line segments 46, 47 lying further close to the rear containment region 27. The second channels 38 are defined between the first and second bonded line segments 29, 30 and the both side edges 20, 21, respectively. The first and the second bonded line segments 46, 47 are spaced from the first and second bonded line segments 29, 30 in the longitudinal direction Y toward the side of the rear containment region 27. Two pairs of the first and second bonded line segments define the double funnel-shaped channel 34 forcing the flow of urine from the rear containment region 27 to be dispersed and thereby to restrict the backflow of urine toward the opening 25.
While a pair of the bonded line segments 46, 47 is formed on the side of the rear containment region 27 according to the present embodiment, it is possible to arrange two or more pairs of the bonded line segments 46, 47 in the longitudinal direction Y or in the transverse direction X. In any case, the number of these bonded line segments 46, 47 may be increased to restrict the backflow of urine in correspondingly improved reliability.

Details of the present invention on the third and fourth aspects thereof will be more fully understood from the description made hereunder based on an embodiment exemplarily described wherein a disposable diaper is taken as an example of the wearing article.

Fig. 11 shows the diaper 101 put on the wearer's body and Fig. 12 is a plan view of the diaper 101 in a flatly developed state after front and rear waist regions having been peeled off each other along both side edges of the diaper 101. In Figs. 11 and 12, the diaper 101 is shown in partially cutaway views for convenience of explanation. In Fig. 12, the contraction of respective elastic members is restricted so that the diaper 101 may be kept in its developed state. Fig. 13 is a sectional view taken along line XIII-XIII in Fig. 12 and Fig. 14 is a sectional view taken along line XIV-XIV in Fig. 12. The diaper 101 includes chassis 102, waist elastic members 103 and leg elastic members 104. The chassis 102 comprises a front waist region 105, a rear waist region 106 and a crotch region 107 extending between these front and rear waist regions 105, 106. These regions 105, 106, 107 are contiguous one to another in a longitudinal direction Y. In addition, the chassis 102 has a longitudinal center line P-P bisecting a dimension of the diaper 1 in a transverse direction X and a transverse center line Q-Q bisecting a dimension of the diaper 1 in the longitudinal direction Y.

The chassis 102 includes an inner liner 108 defining a side of the chassis 102 facing the wearer's body and an outer sheet 109 defining an opposite side of the chassis 102 facing away from the wearer's body. The waist elastic members 103 and the leg elastic members 104 are sandwiched between these liner 108 and outer sheet 109. Both the waist elastic members 103 and the leg elastic members 104 are bonded to at least one of the inner liner 108 and the outer sheet 109. The chassis 102 includes front waist side edges 110, rear waist side edges 111 and crotch side edges 112.

The chassis 102 includes front and rear ends 113, 114 extending in the transverse direction X, i.e., extending orthogonally to the longitudinal direction Y. The crotch side edges 112 are curved in the crotch region 107 toward the longitudinal center line P-P and the leg elastic members 104 extend along the respective crotch side edges 112 and are contractibly attached under tension to the respective crotch side edges 112. The waist elastic members 103 extend along the front and rear ends 113, 114 of the chassis 102 and are contractibly attached under tension and in the transverse direction X to the respective crotch side edges 112. The font waist side edges 110 and the rear waist side edges 111 are joined together to connect these front and rear waist regions 105, 106 with each other and to form a pants-type diaper 101.

A bodily fluid containment structure 115 is attached to the outer side of the outer sheet 109. The containment structure 115 is placed at least in the crotch region 107 and extends into the front and rear waist regions 105, 106 in the longitudinal direction Y.
In the crotch region 107, these inner liner 108 and outer sheet 109 are formed with a through-hole 116 extending through them. Through this through-hole 116, the containment structure 115 is exposed. The through-hole 116 is formed on the side of the front waist region 105 with respect to the transverse center line Q-Q and in alignment with the wearer's external genital. In other words, the wearer's external genital is aligned with the containment structure 115 via the opening 116.

Fig. 15 is a partially cutaway plan view of the containment structure 115 partially cutaway for convenience of explanation. As shown, the containment structure 115 includes rectangular inner and outer sheets 118, 119 both of which are generally the same in size and shape. These inner and outer sheets 118, 119 are bonded to each other along opposite side edges 120, 1121 extending in the longitudinal direction Y and front and rear ends 22, 123 extending in the transverse direction X, respectively. In other words, the inner and outer sheets 118, 119 are bonded to each other along outer peripheries thereof to form an envelope 124. The inner and outer sheets 118, 119 may be formed of liquid-impervious film to make the envelope 124 as a whole liquid-impervious. The inner sheet 118 is formed with an opening 125. Within the envelope 124, front and rear containment regions 126, 127 adapted to contain bodily fluids are defined in front and in the rear of the opening 125 in the longitudinal direction Y and the opposite side edges 120, 121 cooperate with the front and rear ends 122, 123 to define the peripheral edge of the envelope 124.

The inner sheet 118 of the containment structure 115 lies on the side facing the wearer's body and is bonded to the outer sheet 109 of the chassis 102. The opening 125 of the containment structure 115 is formed so as to be aligned with the through-hole 116 extending through the inner liner 108 and the outer sheet 109. A peripheral edge of the opening 125 is bonded to the outer sheet 109 by first bonding means 149 (See Figs. 11 and 12). The first bonding means 149 is formed along the peripheral edge of the opening 125 to prevent the opening 125 and the through-hole 116 from being separated from each other. In consequence, the wearer's external genital aligned with the through-hole 116 is surely aligned with the opening 125 of the containment structure 115 so that discharged urine is reliably guided through the opening 125 into the envelope 124. The through-hole 116 and the opening 125 may be appropriately dimensioned to receive the wearer's external genital. An opening area of the opening 125 is larger than an opening area of the through-hole 116 and therefore a peripheral edge of the through-hole 116 is not aligned with a peripheral edge of the opening 125. In consequence, the wearer's external genital may come in contact with the peripheral edge of the through-hole 116 but not with the peripheral edge of the opening 125. Thus a probability that the peripheral edges of the through-hole and/or the opening come in contact with the wearer's external genital is correspondingly reduced. The probability of such contact reduced in this manner reduces undesirable irritation to the wearer's skin which may cause various types of skin troubles.

Between the inner and outer sheets 118, 119, a backflow barrier sheet 132 is provided so as to overlap and to cover the opening 125 wherein the backflow barrier sheet 132 is formed, for example, by a liquid-impervious film. Between the backflow barrier sheet 132 and the outer sheet 119, a central containing region 151 capable of containing of urine is defined. The backflow barrier sheet 132 has front and rear ends 138, 139 extending in the transverse direction X and side edges 140, 141 extending in the longitudinal direction Y. These front and rear ends 138, 139 and side edges 140, 141 of the backflow barrier sheet 132 lie outside the opening 125 and the backflow barrier sheet 132 is bonded to the inner sheet 118 in a bonded region 128. Outside the bonded region 128, an outer side non-bonded region 131 is defined. The outer side non-bonded region 131 extends between the boned region 128, and the front and rear ends 138, 139 and the side edges 140, 141 of the backflow barrier sheet 132. In the outer side non-bonded region 131, the inner sheet 118 and the backflow barrier sheet 132 merely overlap each other.

The bonded region 128 comprises a plurality of line segments arranged to surround the opening 125 but not to overlap first bonding means 149. Specifically, the bonded region 128 includes first bonded line segments 129 and second bonded line segments 130 both extending obliquely with respect to the longitudinal center line P-P. The first bonded line segments 129 extend obliquely right down and the second bonded line segments 130 extend obliquely right up as viewed in Fig. 15. On the side of the front containment region 126, these first bonded line segments 129 and second bonded line segments 130 are arranged intermittently in the transverse direction X outside the opening 125 as viewed in the longitudinal direction Y. On the side of the front containment region 126, the first bonded line segment 129 cooperates with the adjacent second bonded line segment 130 to define a funnel-shaped rear side channel 133 having its width gradually reduced from the opening 125 toward the front containment region 126. On the side of the rear containment region 127, the first bonded line segment 129 cooperates with the adjacent second bonded line segment 130 to define a funnel-shaped rear side channel 134 having its width gradually reduced from the opening 125 toward the rear containment region 127. Between the opening 125 and the side edges 120, 121, the first bonded line segments 129 and the second bonded line segments 130 are respectively arranged intermittently in the longitudinal direction Y to define lateral side channels 135, respectively.

Between the opening 125 and the first and second bonded line segments 129, 130, an inner side non-bonded region 136 without the bonded region 128 is defined. In other words, the inner sheet 118 and the backflow barrier sheet 132 merely overlap and not bonded to each other along the peripheral edge of the opening 125. The first bonding means 149 is formed so as to overlap the inner side non-bonded region 136.

In the front and rear containment regions 126, 127 and the central containment region 151 are respectively formed with a liquid-absorbent structure 137. In other words, the liquid-absorbent structure 137 extends between opposite side edges 120, 121 as well as between front and rear ends 122, 123 of the envelope 124. The liquid-absorbent structure 137 comprises a liquid-absorbent core formed of superabsorbent resin particles and/or fluff pulp fibers and a liquid-dispersant sheet such as tissue paper with which the liquid-absorbent core is wrapped and is bonded to the outer sheet 119 by second bonding means 150.

In the aforementioned arrangement, upon urination occurring toward the opening 125, the inner sheet 118 and the backflow barrier sheet 132 are spaced from each other under urine pressure and the discharged urine is guided into the inner side non-bonded region 136 and temporarily contained therein. If the wearer is in upright posture, the urine is forced to flow from the inner side non-bonded region 136 downward to the crotch region and the quantity having been temporarily contained flows principally toward the rear side channels 134. The rear side channel 134 is defined by the first and second bonded line segments 129, 130 in a funnel-shape and the urine having been temporarily contained in the inner side non-bonded region 136 is collected by and passing through the rear side channel 134. In the course of passage of urine through the rear side channel 134, the inner sheet 118 and the backflow barrier sheet 132 are spaced from each other to form a tubular channel. The urine having passed through the rear side channel 134 is then guided via outer side non-bonded regions 131 to the rear containment region 127 defined between the inner and outer sheets 118, 119. The urine having been guided into the rear side containment region 127 is absorbed and contained by the liquid-absorbent structure 137.

When the wearer's posture changes, for example, from an upright posture to a side lying posture or to a groveling posture, there is a possibility that the urine having been not absorbed by the liquid-absorbent structure 37 might move from the rear containment region 127 to the central containment region 151 and/or the front containment region 126. However, after the flow of urine has passed through the rear side channel 134, the inner sheet 118 and the backflow barrier sheet 132 having been spaced from each other to define the tubular channel are put again in contact with each other. In consequence, the tubular channel collapses and loses its function as the channel. After a flow of urine has been guided into the front and rear containment regions 126, 127, a sufficient quantity of urine is present also between the outer sheet 119 and the backflow barrier sheet 132 to press the backflow barrier sheet 132 against the inner sheet 118 and thereby to put the backflow barrier sheet 132 in closer contact with the inner sheet 118. As a result, the front and rear channels 133, 134 collapse and lose the function thereof as the channels. This means that the flow of urine moving between the front and rear containment regions 126 and 127 can not smoothly pass through the front and rear channels 133, 134 and the backflow of urine toward the opening 125 through the front and rear channels 133, 134 can be reliably restricted.

In the outer side non-bonded region 131 and the inner side non-bonded region 136 also, the backflow barrier sheet 132 and the inner sheet 118 are put in close contact with each other and thereby make it difficult for urine to pass through these regions 131, 136. Particularly in the outer side non-bonded region 131, the movement of urine to the front and rear channels 133, 134 can be restricted. More specifically, the outer side non-bonded region 131 includes, outside the bonded region 128, a sub-region in which the backflow barrier sheet 132 and the inner sheet 118 come in direct contact with each other. In consideration of this, the area of the backflow barrier sheet 132 may be enlarged to enlarge the outer side non-bonded region 131 and thereby to restrict the movement of urine into the front and rear channels 133, 134 at a correspondingly improved reliability.
Even when the flow of urine moves in the longitudinal direction between the front and rear containment regions 126, 127 in response of a change of the wearer's posture, the flow of urine merely moves between the front and rear containment regions 126, 127 and urine leakage out of the opening can be restricted because not only the inflow of urine into the front and rear channels 133, 134 but also the backflow of urine to the opening 125 can be restricted.

When the backflow barrier sheet 132 is formed of a liquid-impervious film, a slight quantity of urine staying between the backflow barrier sheet 132 and the inner sheet 118 after the flow of urine has passed through a gap defined between these two sheets 132, 118 serves to put these two sheets in close contact with each other. As a result the front and rear side channels 133, 134 defined between these two sheets 132, 118 as well as the outer side non-bonded region 131 and the inner side non-bonded region 136 are closed, making it possible to restrict the urine leakage out of the opening further reliably.

The front and rear side first channels 133, 134 are formed in a funnel-like shape, respectively, so that urine discharged into the opening 125 can be collected and guided into the front and rear containment regions 126, 127. Such arrangement allows a relatively large quantity of urine to be guided into the front and rear containment regions 126, 127 even if the front and rear channels 133, 134 are dimensioned to be relatively narrow. As an additional effect obtained by forming the front and rear channels 133, 134 in a funnel-like shape, these channels 133, 134 force the flow of urine moving from the front and rear containment regions 126, 127 toward the opening 125 to be disperse back. As a result, the movement of urine into the front and rear channels 133, 134 can be reduced and urine leakage out of the opening 125 can be further reliably restricted.

According to the present embodiment, discharged urine can be contained in the bodily fluid containment structure 115 combined with the diaper 101 as the wearing article and there is no possibility that the urine once having been contained in the containment structure 115 might leak out of the structure 115 unless any urine leaks out through the opening. Compared to the diaper including the containment structure 115, urine leakage out of the wearing article can be restricted at the highest reliability. In addition, the separately prepared envelope adapted to receive the discharged urine allows a relatively large quantity of urine to be contained within the envelope and thereby makes it unnecessary to exchange the diaper 1 at short intervals. In other words, the diaper 101 can be maintained put on the wearer's body for a long period.

The inner and outer sheets 118, 119 of the containment structure 115 may be formed, for example, of a liquid-impervious film and the inner liner 108 as well as the outer sheet 109 may be formed, for example, of a liquid-pervious fibrous nonwoven fabric. While the liquid-impervious film is used as materials for the backflow barrier sheet 132 according to the present embodiment, the other widely used sheet materials such as a fibrous nonwoven fabric so far as this sheet is liquid-impervious. However, in order to assure that the inner sheet 118 and the backflow barrier sheet 132 are put in close contact with each other via a slight quantity of urine staying between these two sheets, at least the opposite surfaces thereof facing each other is preferably smooth. Each of these sheets may be formed of a single sheet or two or more sheets laminated together.

While the containment structure 115 is formed with only one opening 125 according to the present embodiment, for example, the rear containment region 127 may be formed with an opening independently of the opening 125. In this case, this additional opening may be used to receive feces. While the bonded region 128 is defined by a plurality of rectilinear line segments according to the present embodiment, the bonded region may comprise a plurality of curved line segments. It is not essential to provide the bonded region 128 in the form of the line segments, and dots or a combination of dots and line segments may be adopted to define the bonded region 128. While the bonded region 128 is defined by the first and second bonded line segments 129, 130 according to the present embodiment, the configuration of the bonded region 128 may be appropriately selected so far as urine backflow from the containment region toward the opening 125 can be prevented. The bonded region 128 may be formed by conditionally used well known means such as adhesives, thermal or ultrasonic bonding techniques.

While the liquid-absorbent structure 137 is formed so as to extend continuously generally over the entire range of the containment structure 115 according to the present embodiment, it is possible, for example, to form this structure 137 intermittently so that this structure is formed in the front and rear containment regions 126, 127 but not in the central containment region 151. However, by forming the central containment region 151 and providing this central containment region 151 also with the liquid-absorbent structure 137, the liquid-absorbent structure 137 to be formed can be bulked and thereby the absorption capacity for bodily fluids such as urine can be improved. While the pullon pants-type diaper is exemplarily illustrated as the wearing article, the present invention is applicable also to a so-called open-type diaper having front and rear waist regions adapted to be joined together along opposite side edges just before the diaper is put on the wearer's body.

Figs. 16 and 17 illustrate the other embodiments of the containment structure 115 characterized in that the inner sheet of the containment structure 115 comprises a first inner sheet 118 lying on the side facing the wearer's body and a second inner sheet 142 lying on the side facing away from the wearer's body. Fig. 16 is a view similar to Fig. 15 and Fig. 17 is a sectional view taken along line XVII-XVII in Fig. 16. The second inner sheet 142 is formed, for example, of a liquid-impervious film generally the same in size as the backflow barrier sheet 132. Specifically, the second inner sheet 142 is contoured by front and rear ends 145, 146 extending in the transverse direction X and opposite side edges 147, 148 extending in the longitudinal direction Y wherein the front and rear ends 145, 146 of the second inner sheet 142 substantially correspond to the front and rear ends 138, 139 of the backflow barrier sheet 132 and the opposite side edges 147, 148 of the second inner sheet 142 substantially correspond to the opposite side edges 140, 141 of the backflow barrier sheet 132. The second inner sheet 142 is formed with an opening 143 and the first and second inner sheets 118, 142 are bonded to each other by bonding means 144 so that the opening 143 may be aligned with the opening 125 of the first inner sheet 118. The bonding means 144 is continuously formed around the opening 143 of the second inner sheet 142.

According to the present embodiment, the bonded region 128 is formed between the second inner sheet 142 and the backflow barrier sheet 132. The bonded region 128 comprises a pair consisting of the first bonded line segment 129 and the second bonded line segment 130 located on the side of the front containment region 126 and a pair consisting of the first bonded line segment 129 and the second bonded line segment 130 located on the side of the rear containment region 127. The first and second bonded line segments 129, 130 lying on the side of the front containment region 126 cooperate with each other to define the front side channel 133 and the first and second bonded line segments 129, 130 lying on the side of the rear containment region 127 cooperate with each other to define the rear side channel 134. On both sides of the opening 143 as viewed in the transverse direction X, the first and second bonded line segments 129, 130 cooperate with each other to define lateral channels 135. '

With such arrangement, urine discharged toward the opening 125 of the first inner sheet 118 is guided via the opening 143 of the second inner sheet and the inner side non-bonded region 136 to the front and rear channels 133, 134 and received by the front and rear containment regions 126, 127 via the outer side non-bonded region 131. The front and rear side channels 133, 134 as well as the lateral channels 135 each formed between the second inner sheet 142 and the backflow barrier sheet 132 collapse and lose the function thereof as the channels after the flow of urine has passed through these channels since the second inner sheet 142 and the backflow barrier sheet 132 are put in contact again with each other. Therefore, there is no anxiety that the urine once having been guided into the front and rear containment regions 126, 127 might back flow toward the opening 125 via these channels. The second inner sheet 142 and the first inner sheet 118 are bonded to each other along the peripheral edges of the openings 143, 125 by the bonding means 144 and obviously there is no possibility that urine leak might occur out of opposed surfaces of these sheets.

The second inner sheet 142 is bonded to the first inner sheet 118 merely along the peripheral edge of the opening 143 and in the remaining region, i.e., in the vicinity of the front and rear ends 145, 146 and the opposite side edges 147, 148 of the second inner sheet, the second inner sheet 142 can be spaced from the first inner sheet 118. Therefore, the second inner sheet 142 can follow the movement of the backflow barrier sheet 132. Even when the backflow barrier sheet 132 moves toward the outer sheet 119, there is no possibility that the second inner sheet 142 and the backflow barrier sheet 132 might be spaced from each other. This means that the front and rear channels 133, 134 as well as the lateral channels 135 defined between these sheets 142, 143 can be maintained in closed state. In this way, these channels 133 through 135 can be kept in state of so-called *functio laesa* and backflow of urine through the channels can be further reliably prevented.

### IDENTIFICATION OF REFERENCE NUMERALS USED IN THE DRAWINGS

- 1: diaper
- 2: chassis
- 5: front waist region
- 6: rear waist region
- 7: crotch region
- 15: bodily fluid containment structure
- 16: through-hole
- 18: inner sheet
- 19: outer sheet
- 20: side edges
- 21: side edges
- 24: envelope
- 25: opening
- 26: front side containment region (first non-bonded region)
- 27: rear side containment region (first non-bonded region)
- 28: bonded region
- 33: front side first channels
- 34: rear side first channels
- 36: second non-bonded region
- 38: second channels
- 40: front side liquid-absorbent structure
- 41: rear side liquid-absorbent structure
- 101: diaper
- 102: chassis
- 105: front waist region
- 106: rear waist region
- 107: crotch region
- 115: bodily fluid containment structure
- 116: through-hole
- 118: inner sheet (first inner sheet)
- 119: outer sheet
- 120: side edges
- 121: side edges
- 124: envelope
- 125: opening
- 126: front side containment region
- 127: rear side containment region
- 128: bonded region
- 131: outer side non-bonded region
- 132: backflow barrier sheet
- 133: front side channels
- 134: rear side channels
- 136: inner side non-bonded region
- 137: liquid-absorbent structure
- 142: second inner sheet
- 151: central containment region

## Claims

1. A bodily fluid containment structure having a longitudinal direction extending rearward from a front of the wearer's body, a transverse direction being orthogonal to said longitudinal direction, comprising a liquid impervious inner sheet facing the wearer's body and a liquid-impervious outer sheet facing away from the wearer's body wherein said inner and outer sheets cooperate with each other to form an envelope, said inner sheet is formed with an opening allowing bodily fluids to flow into said envelope and a bodily fluid containment region is formed between said inner and outer sheet as said inner and outer sheets are spaced from each other by inflow of bodily fluids thereinto, said bodily fluid containment structure being **characterized in that**:
a bonded region substantially bonding said inner and outer sheets is formed between a peripheral edge of said opening and a peripheral edge of said envelope so that said bonded region forms first channel allowing bodily fluids to move between said opening and said containment region; and
said containment region is defined between said first channel and a peripheral edge of said envelope.

2. A bodily fluid containment structure comprising a chassis having a longitudinal direction, a transverse direction, a side facing the wearer's body and a side facing away from the wearer' s body and further comprising a front waist region, a rear waist region and a crotch region extending between said front and rear waist regions wherein these regions are contiguous one to another in said longitudinal direction, said wearing article being **characterized in that**:
said wearing article comprises the bodily fluid containment structure defined in Claim 1 at least in said crotch region; and
said opening of said bodily fluid containment structure lies in said crotch region.

3. A bodily fluid containment structure having a longitudinal direction extending rearward from a front of the wearer's body, a transverse direction being orthogonal to said longitudinal direction, comprising a liquid-impervious inner sheet facing the wearer's body and a liquid-impervious outer sheet facing away from the wearer's body wherein said inner and outer sheets cooperate with each other to form an envelope, said inner sheet is formed with an opening allowing bodily fluids to flow into said envelope and a bodily fluid containment region is formed between said inner and outer sheet as said inner and outer sheets are spaced from each other by inflow of bodily fluids thereinto, said bodily fluid containment structure being **characterized in that**:
a backflow barrier sheet covering said opening is provided between said inner and outer sheet; and
said backflow barrier sheet is intermittently bonded to said inner sheet by means of a bonded region so that said containment region is formed between said backflow barrier sheet and said outer sheet also.

4. A wearing article comprising a chassis having a longitudinal direction and a transverse direction, a side facing the wearer's body and a side facing away from the wearer's body, a front waist region, a rear waist region and a crotch region extending between said front and rear waist regions wherein these regions are contiguous one to another in said longitudinal direction, said wearing article being **characterized in that**:
said wearing article includes the bodily fluid containment structure defined in Claim 3 at least in said crotch region; and
said opening of said bodily fluid containment structure lies in said crotch region.
